# EUROPEAN PATENT SPECIFICATION

(11) **EP 2 139 838 B1**
(45) Date of publication and mention of the grant of the patent: **28.03.2012**
(21) Application number: 07858233.5
(22) Date of filing: 28.12.2007
(51) Int. Cl.: C07C 51/09, C07C 65/26, C07C 67/343, C07C 69/00, C07F 5/02

(54) **A PROCESS FOR THE PREPARATION OF 6-[3-(1-ADAMANTYL)-4-METHOXYPHENYL]-2-NAPHTOIC ACID**
VERFAHREN ZUR HERSTELLUNG VON 6-[3-(1-ADAMANTYL)-4-METHOXYPHENYL]-2-NAPHTOESÄURE
PROCÉDÉ DE PRÉPARATION D'ACIDE 6-[3-(1-ADAMANTYL)-4-MÉTHOXYPHÉNYL]-2-NAPHTOÏQUE

(30) Priority: 29.12.2006 LV 060150
(43) Date of publication of application: 06.01.2010
(73) Proprietor: Grindeks, a joint stock company, Riga 1057 (LV)
(72) Inventor: Kalvinsh, Ivars, 5052 Ikskile (LV); Chernobrovijs, Aleksandrs, 2114 Olaine (LV); Tribulovich, Vyacheslav, Sankt-Peterburg 197022 (RU); Labeish, Vladimir, Sankt-Peterburg 198332 (RU)
(86) International application number: PCT/EP2007/064647
(87) International publication number: WO 2008/080992

(56) References cited:
- EP-A- 1 707 555
- WO-A-01/56563

## Description

The present invention concerns a process for the preparation of 6-[3-(1-adamantyl)-4-methoxyphenyl]-2-naphthoic acid (adapalene). Adapalene is used for treating *acne vulgaris* [Waugh, J.; Noble, S.; Scott, L. Spotlight on adapalene in acne vulgaris. J. Am. J. Clin. Dermatol. 2004, 5 (5), 369-371; Jain, S. Topical tretinoin or adapalene in acne vulgaris: an overview. J. Dermatol. Treat. 2004, 15 (4), 200-207.] This substance also possesses antitumour activity [WO 2001/056563, Galderma Research (USA)]. Therefore a convenient process for the preparation of adapalene is desirable.

A method for preparation of 6-[3-(1-adamantyl)-4-methoxyphenyl]-2-naphthoic acid is known since 1986 [Shroot, B.; Eustache, J.; Bernardon, J.-M. EP 199636; Benzonaphtalene derivatives and compositions. US 4717720 (1988)]. It is a multi-step process, involving the following steps:
a) alkylation of 4-bromophenol with adamantanol-2 to yield 2-(1-adamantyl)-4-bromophenol;
b) O-alkylation of 2-(1-adamantyl)-4-bromophenol with Mel to yield 2-(1-adamantyl)-4-bromoanisole;
c) preparation of Grignard's reagent from 2-(1-adamantyl)-4-bromoanisole, transformation thereof into a zinc derivative and Ni-catalyzed coupling with methyl 6-bromo-2-naphthenoate to yield methyl-6-[3-(1-adamantyl)-4-methoxyphenyl]-2-naphthenoate;
d) basic hydrolysis of methyl-6-[3-(1-adamantyl)-4-methoxyphenl]-2-naphthenoate to yield 6-[3-(1-adamantyl)-4-methoxyphenyl]-2-naphthoic acid.

This process has a number of disadvantages:
1) purification of 2-(1-adamantyl)-4-bromoanisole and methyl-6-[3-(1-adamantyl)-4-methoxyphenyl]-2-naphthenoate requires using column chromatography;
2) preparation of Grignard's reagent from 2-(1-adamantyl)-4-bromoanisole, transformation thereof into a zinc derivative and Ni-catalyzed coupling with methyl 6-bromo-2-naphthenoate to yield methyl-6-[3-(1-adamantyl)-4-methoxyphenyl]-2-naphthenoate in step (c) is difficult to control on industrial scale and does not give stable yields.

A prospective method for manufacturing adapalene appears to be the employment of Suzuki-Miyaura reaction [Miyaura, N.; Suzuki, A. Palladium-catalyzed cross-coupling reactions of organoboron compounds. Chem. Rev. 1995, 95 (7), 2457-2483; Suzuki, A. Recent advances in the cross-coupling reactions of organoboron derivatives with organic electrophiles, 1995-1998. J. Organomet. Chem. 1999, 576, 147-168; Kotha, S.; Lahiri, K.; Kashinath, D., Recent applications of the Suzuki-Miyaura cross-coupling reaction in organic synthesis. Tetrahedron 2002, 58, 9633-9695].

Using Suzuki-Miyaura reaction in this process should provide for efficient coupling of two known compounds - 3-(1-adamantyl)-4-methoxyphenylboronic acid or suitable derivative thereof with a suitable derivative of methyl-6-substituted-2-naphthenoate.

In 2006 a process was published (Castaldi, G.; Allegrini, P.; Razezetti, G.; Ercoli, M.; A process for the preparation of adapalene, EP 1707555 A), using for the synthesis of adapalene an already known (Zim, D.; Lando, V.R.; Dupont, J.; Montiero, A.L. Org. Lett. 2001, 3, 3049) version of the Suzuki-Miyaura reaction for coupling 3-(1-adamantyl)-4-methoxyphenylboronic acid with tosyloxy-2-naphthenoate in presence of Ni(II) salt and phosphine ligands:

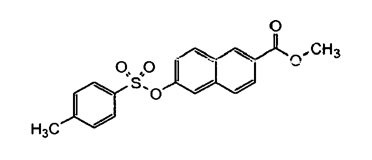

Unfortunately the patent does not disclose either the preparation of 3-(1-adamantyl)-4-methoxyphenylboronic acid or the method for preparing tosyloxy-2-naphthenoate. The known processes for preparing 3-(1-adamantyl)-4-methoxyphenylboronic acid do not provide for inexpensive manufacturing in stable and fair yields. Our attempts to repeat the method of Castaldi demonstrated that the coupling catalyzed by Ni(II)/phosphine complex is an unstable process with variable preparative yields.

WO 01/56563 discloses the hydrolysis of derivatives of adapalene methyl esters using potassium hydroxide in n-butanol.

Therefore the objective of this invention was to develop an alternative process based on Suzuki-Miyaura reaction for manufacturing of 6-[3-(1-adamantyl)-4-methoxyphenyl]-2-naphthoic acid characterized by stable and high yields, easily scaleable up and suitable for industrial application.

Since the most important and difficult to produce intermediate in this process is the 3-(1-adamantyl)-4-methoxyphenylboronic acid we had to develop a convenient method for preparing thereof.

A method for synthesis of 3-(1-adamantyl)-4-methoxyphenylboronic acid starting from 2-(1-adamantyl)-4-bromoanisole *via* the corresponding Li-derivative is known: 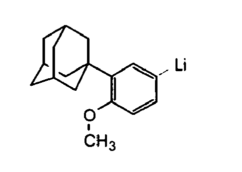 [Sarshar, S. Novel therapeutic agents for the treatment of cancer, metabolic diseases and skin disorders. WO 2005/108338 A1]). The main disadvantage of this method is the low yield of the end product, not exceeding 12%. It is important to notice that both the metallation and transmetallation reactions are conducted at -78 °C that considerably complicates the realization of this process on industrial scale.

Therefore it was necessary to develop a preparative method for preparation of 3-(1-adamantyl)-4-methoxyphenylboronic acid and derivatives thereof, suitable in the synthesis of adapalene, that could be appropriate for industrial application, giving high yields at temperatures that are convenient for industrial processes.

It is possible to prepare the 3-(1-adamantyl)-4-methoxyphenylboronic acid and its derivatives useful for the synthesis of adapalene from 2-(1-adamantyl)-4-bromoanisole by transforming it into a modified Grignard's reagent 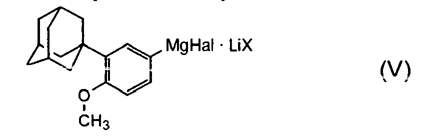 obtained by conducting the reaction in a selected solvent, e.g., tetrahydrofuran and using simultaneously two activators of the reaction - lithium chloride and dibromoethane.

The molar proportion of LiCl and 2-(1-adamantyl)-4-bromoanisole in the preparation of the Grignard's reagent may be from 0.5 to 3.0, but the best yields are obtained with the excess of LiCl.

The optimal proportion of these reagents is from 1.2 to 1. A further increasing of LiCl rate up to 3:1 does not improve the yield.

Various Li salts can be used in the synthesis of the Grignard's reagent (V) from 2-(1-adamantyl)-4-bromoanisole, e.g., LiBr, Lil, Li₂SO₄, LiClO₄, LiBF₄, but the best results are provided by using LiCl.

The temperature in the reaction medium can be from about minus 70 °C to plus 65 °C, but it is more convenient to conduct this process at the boiling temperature of tetrahydrofuran.

Instead of 2-(1-adamantyl)-4-bromoanisole other 2-(1-adamantyl)-4-haloanisoles can be used, e.g., chloro or iodo derivative.

3-(1-Adamantyl)-4-methoxyphenylboronic acid can be prepared from the aforementioned Grignard's reagent even between 0 °C - +5 °C. It is known that similar arylboronic acids can be prepared only at much lower temperatures, e.g., -70 °C. [Seaman, W.; Jonson, J.R. Derivatives of phenylboric acid, their preparation and action upon bacteria. J. Am. Chem. Soc. 1931, 53 (2), 711-723; Bean, F.R.; Jonson, J.R. Derivatives of phenylboric acid, their preparation and action upon bacteria. II. Hydroxyphenylboric acids. J. Am. Chem. Soc. 1932, 54 (11), 4415-4425. Cladingboel, D.E. A large-scale synthesis of [4-(2-(2H)-tetrahydropyranyloxy)phenyl]boronic acid. Org. Process Res. Dev.,2000, 4 (3), 153-155].

Lowering the temperature at the coupling step does not increase the yield of 3-(1-adamantyl)-4-methoxyphenylboronic acid.

The Grignard's reagent can be transformed into the derivative of 3-(1-adamantyl)-4-methoxy-phenylboronic acid by reacting with trialkylborates, preferably with trimethylborate. In this reaction trimethylborate can be substituted with other electrophilic trialkylborates, but usually it leads to lower yields of 3-(1-adamantyl)-4-methoxyphenylboronic acid.

The 3-(1-adamantyl)-4-methoxyphenylboronic acid is not a very stable compound. The instability of 3-(1-adamantyl)-4-methoxy-phenylboronic acid is demonstrated by its NMR spectrum, where a varying proportion of signals from its cyclic trimer (boroxine) is observed For the formation of the C-C- link it is essential to have one of the coupling components, namely 3-(1-adamantyl)-4-methoxyphenylradical, substituted by a boron-containing functional group, e.g.: 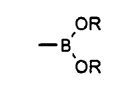 wherein R is H, alkyl- or cycloalkylgroup.

Investigating the problems of stabilizing 3-(1-adamantyl)-4-methoxy-phenylboronic acid we synthesized a series of alkyl 3-(1-adamantyl)-4-methoxyphenylborates that can be conveniently prepared by the action of Grignard's reagent on trialkylborates. 3-(1-Adamantyl)-4-methoxyphenylboronic acid can be easily transformed into a corresponding cyclic ester, e.g., pinacol ester, by heating 3-(1-adamantyl)-4-methoxyphenylboronic acid with 2,3-dimethyl-2,3-butanediol (pinacol) in toluene with removal of water by azeotropic distillation. Similarly are prepared the cyclic esters of ethylene glycol and 2,2-dimethyl-1,3-propanediol.

Thus we have prepared esters of 3-(1-adamantyl)-4-methoxyphenylboronic acid that are useful in the synthesis of adapalene.

The adapalene synthesis of the present invention, starting from the easily accessible 2-(1-adamantyl)-4-bromoanisole, contains the following steps:
A) reproducible and scaleable method for preparing 3-(1-adamantyl)-4-methoxyphenylboronic acid via Grignard's reagent produced in high yield from 2-(1-adamantyl)-4-bromoanisole at 0-5 °C in presence of LiCl and dibromoethane;
B) industrially applicable catalytic coupling of 3-(1-adamanty!)-4-methoxy-phenylboronic acid or derivatives thereof in Suzuki-Miyaura reaction with 6-halonaphthoic ester, yielding 6-[3-(1-adamantyl)-4-methoxyphenyl]-2-naphthoic ester, with a following its basic hydrolysis to 6-[3-(1-adamantyl)-4-methoxyphenyl]-2-naphthoic acid in 1,2-propylene glycol solution, that allows the separation of the final product by known methods.

Instead of methyl-6-bromo-2-naphhenoate also other esters of 6-halo-2-naphthoic acids can be applied in the coupling reaction, e.g., esters of 6-chloro or 6-iodo-2-naphthoic acid. Likewise said 6-halo-2-naphthoic acid can be used either as a free acid or its Li, Na or K salt.

Likewise the 6-halo-2-naphthoic esters used in the coupling reaction can be formed from alcohols, containing from 1 to 10 carbon atoms, both straight chain and branched.

Further the 6-halo-2-naphthoic esters can also be formed from alicyclic alcohols, containing 3, 4, 5 or 6 carbon atoms in the cycle and optionally substituted in the ring. Further 6-halo-2-naphthoic esters, useful for the method are alkoxymethyl- or aryloxymethylesters with a general formula of the ester group -COO-CH₂OR, wherein R denotes alkyl group, e.g., methyl-, ethyl-, propyl-, isopropyl-, butyl-, isobutyl-, pentyl- or branched pentylgroup, as well as the aryl containing oxymethylgroup, e.g., phenoxymethyl- or benzyloxymethylgroup.

The proposed intermediates can be represented by the following general formula: 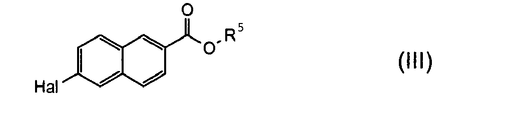 wherein R⁵ is H, K, Na, Li, aryl, C₁-C₁₀ straight, branched or cyclic alkyl, CH₂OC₁-C₅alkyl, and Hal is Cl, Br or I.

As the second component of the coupling reaction the process of the present invention provides for using various derivatives of 3-(1-adamantyl)-4-methoxyphenylboronic acid of general formula: 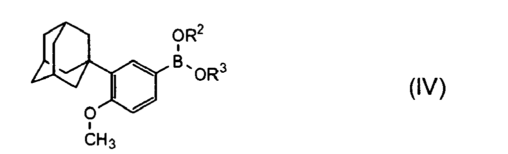 wherein R² and R³ independently of each other is H, K, Na, Li, straight, branched or cyclic C₁-C₁₀ alkyl or aryl.

We have discovered that coupling of 3-(1-adamantyl)-4-methoxy-phenylboronic acid derivative with 6-halo-2-naphthoic ester can be catalyzed by various agents, including basic agents like Li, Na, K or Sr carbonate, acetate or phosphate.

Differently from the known Castaldi process, that employs catalysis by Ni(II) salts and phosphine ligands, we have found that high and reproducible yields in this reaction are provided by palladium-containing catalysts or Pd(0) with or without phosphine ligands, e.g., tetrakis(triphenylphosphine)palladium(0). The most convenient sources of palladium Pd(0) are palladium acetate and tris(dibenzylydeneacetone)-dipalladium(0). Our investigations show that the following phosphine ligands are useful for catalysis of the coupling: 2-dicyclohexyphosphino-2',6'-dimethoxybiphenyl, 2-dicyclohexylphosphino-2'-methylbiphenyl, 2-dicyclohexylphosphino-2',6'-dimethylbiphenyl, 2-dicyclohexylphosphinobiphenyl, 2-di-tert-butylphosphino-2'-methylbiphenyl, 2-di-tert-butylphosphino-2',6'-dimethylbiphenyl, 2-di-tert-butylphosphinobiphenyl, 2,4,6-tri-iso-propyl-2'-biphenylylphosphinobiphenyl, diphenylphosphinoferrocene, triphenylphosphine, tricyclohexylphosphine, as well as tri-tert-butylphosphine, although the best results are obtainable using the 2-dicyclohexylphosphino-2',6'-dimethoxybiphenyl ligand.

The amount of the catalyst depends on the structure thereof and varies within the range from 0.0005 to 0.1 mol for one mol of the 3-(1-adamantyl)-4-methoxyphenylboronic acid derivative used. In most cases the optimal concentration of the catalyst is about 0.005 mol for one mol of the boronic acid derivative. The reaction can be brought about in a suitable organic solvent or mixture thereof as well as in the mixture thereof with water. The reaction temperature depends on the components and catalyst used and is between 20 °C and 100 °C.

The present invention is explained but not limited by the following examples.

### Step A. 2-(1-adamantyl)-4-bromoanisole

### Example 1 (not according to the invention)

In a 1 L flask equipped with a stirrer, a reflux condenser and a dropping funnel, a mixture of 50 g (0.33 M) 1-adamantanol, 68 g (45.5 mL, 0.36 M) 4-bromoanisole and 500 mL of methylene chloride is prepared. The mixture is stirred until dissolved and then 35 g (19 mL, 0.36 M) of sulfuric acid added during one hour. The reaction mixture is stirred for 4 hours, 200 mL of water added, stirred for another 10 min, transferred to a separating funnel and the organic layer collected. It is neutralized by two portions of 100 mL 10% sodium carbonate solution. Methylene chloride is distilled off completely, the residue dissolved in 300 mL of ethyl acetate, filtered, concentrated to 200 mL and left to crystallize at about 0 °C for 16 h. The crystalls are filtered off, washed by 50 mL of cold ethyl acetate and dried at 100 °C for one hour. 2-adamantyl-4-bromoanisole, 76 g (72%) with m.p. 140-141°C is obtained.
NMR (500 MHz, DMSO D₆)
δ: 1.75 (s, 6H), 2.03 (s, 9H), 3.81 (s, 3H), 6.82 (d, 1H, J = 8 Hz), 7.17 (s, 1H), 7.23 (d, 1H, J = 8 Hz).
Scaling the synthesis up 10 times did not change either the yield or quality of the 2-(1-adamantyl)-4-bromoanisole.

### Step B. 3-(1-adamantyl)-4-methoxyphenylboronic acid

### Example 2 (not according to the invention)

In a 1 L flask equipped with a stirrer, a reflux condenser and a dropping funnel, a mixture of 8 g (0.33 M) magnesium filings and 200 mL of dry tetrahydrofuran is stirred. 8 g (0.19 M) of pulverised anhydrous lithium chloride is added. The air in the flask is displaced by argon and all further operations performed under a slight stream of this inert gas. Under intense stirring 11 g (5 mL, 0.06 M) of 1,2-dibromoethane is added in one portion. After the vigorous reaction had subsided, the hot reaction mixture is treated with a solution of 50 g (0.16 M) 2-(1-adamantyl)-4-bromoanisole in 400 mL tetrahydrofuran with such speed that a slight reflux is supported. After adding the solution of 2-(1-adamantyl)-4-bromoanisole the reaction mixture is stirred and heated to slow reflux for another 30 min. The stirring is discontinued and the Grignard's reagent thus obtained is decanted from the residual magnesium into a conical flask, flushed in advance with argon, closed with a ground stopper and kept at 0 °C for 2 h.

In a 1 L flask equipped with a stirrer, a reflux condener and a dropping funnel a mixture of 33 g (36 mL, 0.35 M) of trimethylborate and 100 mL of dry tetrahydrofuran is prepared. The solution thus obtained is cooled to 0 - +5°C (by ice water) and under vigorous stirring treated with the previously prepared Grignard's reagent within 10 min. The reaction mixture is left overnight (16 h) in the refrigerator at 0 - +5°C. The reaction mixture is decomposed with a solution of 50 mL of hydrochloric acid in 50 mL of water with vigorous stirring without external cooling. The mixture is transferred to a separating funnel and the layers separated. To the water layer 200 mL of water is added and extracted twice with 100 mL of diethyl ether. The pooled organic solutions are dried on anhydrous sodium sulfate, the solvents removed under vacuo at about 50 °C in the water bath. The residue is treated with 200 mL of ethyl acetate and left in the refrigerator overnight. The precipitate is filtered off and dried at 100 °C. 3-(1-adamantyl)-4-methoxyphenylboronic acid, 30 g (68%) with m.p. ~300°C is obtained.

### Example 3 (not according to the invention)

Reaction is performed as described in Example 2, using 81 g (94 mL, 0.35 M) of tributylborate as the electrophile. 3-(1-Adamantyl)-4-methoxyphenylboronic acid, 28 g (56%) is obtained.

### Example 4 (not according to the invention)

Reaction is performed as described in Example 2, using 66 g (81 mL, 0.35 M) of triisopropylborate. 3-(1-Adamantyl)-4-methoxyphenyl-boronic acid, 23 g (51%) is obtained.

### Example 5 (not according to the invention)

Reaction is performed as described in Example 2. The Grignard's reagent is produced with 6.8 g (0.16 M, 1 eq.) LiCl. 3-(1-Adamantyl)-4-methoxyphenylboronic acid, 21.2 g (48%) is obtained.

### Example 6 (not according to the invention)

Reaction is performed as described in Example 2. Grignard's reagent is produced with addition of 0.7 g (0.016 M, 0.1 eq.) of LiCl. 3-(1-Adamantyl)-4-methoxyphenylboronic acid, 3.1 g (7%) is obtained.

### Example 7 (not according to the invention)

Reaction is performed as described in Example 2. Grignard's reagent is produced without adding LiCl. 3-(1-Adamantyl)-4-methoxyphenylboronic acid, 1.3 g (3%) is obtained.

### Example 8 (not according to the invention)

Reaction is performed as described in Example 2. Grignard's reagent is produced with addition of 13.6 g (0.32 M, 2 Eq) of LiCl. 3-(1-Adamantyl)-4-methoxyphenylboronic acid, 28 g (63%) is obtained.

### Example 9 (not according to the invention)

Reaction is performed as described in Example 2. Grignard's reagent is produced with addition of 20.4 g (0.48 M, 3 Eq) of LiCl. 3-(1-Adamantyl)-4-methoxyphenylboronic acid, 27 g (62%) is obtained.

### Example 10 (not according to the invention)

Reaction is performed as described in Example 2. The interaction of 3-(1-adamantyl)-4-methoxyphenylmagnesium bromide solution with trimethylborate is performed at -70°C. 3-(1-Adamantyl)-4-methoxyphenylboronic acid, 30.6 g (69%) is obtained.

Scaling the synthesis up 6 times under the optimal reaction conditions did not lower the yield of 3-(1-adamantyl)-4-methoxyphenylboronic acid.

Since the NMR spectrum of 3-(1-adamantyl)-4-methoxyphenyl-boronic acid displays the combined spectra of acid and its cyclic trimer (boroxine) in varying proportions, the identification of the product was performed after the cyclic ester of pinacol was produced.

### Example 11 (not according to the invention)

Into a 1 L flask equipped with Dean-Stark apparatus 100 g (0.35 M) of 3-(1-adamantyl)-4-methoxyphenylboronic acid, 45.5 g (0.39 M) 2,3-dimethyl-2,3-butanediol (pinacol) and 500 mL of toluene was introduced. The mixture was refluxed for 2 hours, while the theoretical quantity of water was collected in the Dean-Stark trap (~13 mL). After the completion the solvent was removed in vacuo at 60 °C in the water bath. The residue was dissolved in 200 mL of ethyl acetate and left for 16 h at about 0 °C. The precipitate was filtered off, washed with a minimal volume of cold ethyl acetate and dried at 80 °C. 3-(1-Adamantyl)-4-methoxyphenylboronic acid pinacol ester, 108 g (84%) with m.p. 162-164 °C is obtained.
NMR (500 MHz, DMSO D₆)
δ: 1.30 (s, 12H), 1.76 (s, 6H), 2.07 (s, 3H), 2.09 (s, 6H), 3.85 (s, 3H), 6.83 (d, 1H, J = 8 Hz), 7.49 (s, 1H), 7.50 (d, 1H, J = 8 Hz).

With ethylene glycol as alcohol, the yield of the ester was 76%; the yield of 2,2-dimethyl-1,3-propanediol ester was 82%. The gas chromatography data give the yield of esters around 98-100%. The lower preparative yield results from losses in the recrystallization.

Scaling the synthesis up 10 times under the optimal reaction conditions did not lower the yield and quality of the end product.

### Step C. Catalytic coupling of 3-(1-adamantyl)-4-methoxyphenyl-boronic acid derivatives with 6-bromo-2-naphtoic acid esters according to Suzuki-Miyaura.

### Example 12 (not according to the invention)

Into a 1 L flask with stirrer and reflux condenser 0.25 g (0.5 mol%) of tris(dibenzylydeneacetone)dipalladium(0) [Pd₂(dba)₃] was dissolved in 500 mL of thetrahydrofuran, then 1 g (0.5 mol%) of 2-dicyclohexyl-phosphino-2',6'-dimethoxybipheyl (Sphos) added and the resulting solution stirred under a slight stream of argon.

To the reaction mixture was added 30 g (0.105 M) of 3-(1-adamantyl)-4-methoxyphenylboronic acid and 25 g (0.094 M) of methyl 6-bromo-2-naphthenoate and stirred until dissoved. The final component is a solution of 30 g (0.28 M) of sodium carbonate in 150 mL of water, prepared in advance, that is added to the reaction mixture in one portion.

The process is carried on under vigorous stirring and reflux under a slight stream or argon.

After the completion the reaction mixture is cooled to room tempearture and filtered without separating the layers. The precipitate constitutes the main portion of the product. The rest of the product is obtained by concentrating the organic layer to ¼, cooling and filtering off the precipitate. The raw product is dissolved in 200 mL of dimethylacetamide, filtered hot, reduced to about 150 mL and left for 16 h at room temperature for crystallization. The voluminous crystals are filtered off and dried at 150-180 °C for 2h. Methyl 6-[3-(1-adamantyl)-4-methoxyphenyl]-2-naphthenoate, 35.2-36.8 g (88-92%) with m.p. 222-225 °C is obtained.
NMR (500 MHz, DMSO D₆)
δ: 1.79 (s, 12H), 2.10 (s, 3H), 2.16 (s, 6H), 3.90 (s, 3H), 3.94 (s, 3H), 6.99 (d, 1H, J = 8 Hz), 7.51 (s, 1H), 7.52 (d, 1H, J = 8 Hz), 7.78 (d, 1H, J = 9 Hz), 7.94 (d, 1H, J = 9 Hz), 7.98 - 8.02 (m, 3H), 8.55 (s, 1H).

Replacing 3-(1-adamantyl)-4-methoxyphenylboronic acid with its pinacol or ethylene glycol ester, or replacing tris(dibenzylydeneacetone)-dipalladium(0) [Pd₂(dba)₃] with palladium acetate (Pd(OAc)₂), or using potassium carbonate or potassium phosphate as the basic agent does not substantially change the yield of methyl 6-[3-(1-adamantyl)-4-methoxyphenyl]-2-naphtenoate.

### Example 13 (not according to the invention)

Reaction is performed as described in Example 12. As the boronic acid component 3-(1-adamantyl)-4-methoxyphenylboronic acid ethylene glycol ester is used, 33 g (0.105 M), as the basic agent - 41 g (0.3 M) of potassium carbonate solution in 200 mL of water. Methyl 6-[3-(1-adamantyl)-4-methoxyphenyl]-2-naphthenoate, 34.5 g (86%) is obtained.

### Example 14 (not according to the invention)

Reaction is performed as described in Example 12. As the boronic acid component 3-(1-adamantyl)-4-methoxyphenylboronic acid pinacol ester is used, 39 g (0.105 M), as the Pd[0] source - palladium acetate, 0.11 g (0.5 mol%). As the basic agent the solution of 60 g (0.28 M) of potassium phosphate in 200 mL of water is used. Methyl 6-[3-(1-adamantyl)-4-methoxyphenyl]-2-naphthenoate, 35 g (87%) is obtained.

A gradual increase of the quantity of catalyst up to 5 mol% leads to the shortening of the reaction time to half-hour, but does not influence the yield of the end product. Reducing the quantity of the catalyst to 0.05 mol% leads to the increase of reaction time to 16 hours and drops the yield of methyl 6-[3-(1-adamantyl)-4-methoxyphenyl]-2-naphthenoate to 76%.

Scaling up the synthesis under the optimal reaction conditions did not lower the yield of methyl 6-[3-(1-adamantyl)-4-methoxyphenyl]-2-naphthenoate.

Using as a catalyst freshly prepared tetrakis(triphenyl-phosphine)palladium(0) Pd[P(Ph)₃]₄ under the optimal reaction conditions, the yield of the end product was lower, both with 3-(1-adamantyl)-4-methoxyphenylboronic acid and 3-(1-adamantyl)-4-methoxyphenylboronic acid pinacol ester.

### Example 15 (not according to the invention)

Reaction is performed as described in Example 12. As the catalyst a freshly prepared tetrakis(triphenylphosphine)palladium(0) Pd[P(Ph)₃]₄ 0.6 g (0.5 mol%) is used. Methyl 6-[3-(1-adamantyl)-4-methoxyphenyl]-2-naphthenoate, 27.2 g (68%) is obtained.

### Example 16 (not according to the invention)

Reaction is performed as described in Example 12. As the boronic acid component 3-(1-adamantyl)-4-methoxyphenylboronic acid pinacol ester is used, 39 g (0.105 M). As the catalyst a freshly prepared tetrakis(triphenylphosphine)palladium(0) Pd[P(Ph)₃]₄ 0.6 g (0.5mol%) is used. Methyl 6-[3-(1-adamantyl)-4-methoxyphenyl]-2-naphthenoate, 23 g (57%) is obtained.

Methyl 6-[3-(1-adamantyl)-4-methoxyphenyl]-2-naphthenoate can be prepared without separation and purification of 3-(1-adamantyl)-4-methoxyphenylboronic acid, produced in Step B.

### Example 17 (not according to the invention)

Into a 2 L flask equipped with a stirrer, a reflux condenser and a dropping funnel 18 g (0.75 M) of magnesium filings are introduced and 400 mL of dry tetrahydrofuran added. Pulverized anhydrous lithium chloride, 17 g (0.4 M) is added. All operations are performed under a slight stream of argon. Under vigorous stirring 22 g (10 mL, 0,12 M) of 1,2-dibromoethane is added. After the end of the reaction, to the reaction mixture 100 g (0.31 M) of 2-(1-adamantyl)-4-bromoanisole dissolved in 700 mL of dry tetrahydrofuran is added with such spead that the reaction mixture is kept under slight reflux. After the adding of all 2-(1-adamantyl)-4-bromoanisole solution, the reaction mixture is refluxed for another 30 min. The stirring is discontinued and the solution of Grignard's reagent is decanted from the residual magnesium into a conical flask, flushed with argon in advance, and kept closed at 0 °C for two hours.

In a 2 L flask equipped with a stirrer, a reflux condenser and a dropping funnel a solution of 70 g (76 mL, 0.67 M) trimethylborate in 200 mL of dry tetrahydrofuran is prepared, the solution cooled to 0 - +5°C and under vigorous stirring the solution of the Grignard's reagent is added within 10 min. The resulting mixture is left for 16 h at about 0 °C in the refrigerator. The decomposition is performed by slow adding under vigorous stirring of 120 mL of saturated aqueous ammonium chloride solution. The organic layer is decanted from the inorganic salts and the residue washed with additional 120 mL of tetrahydrofuran.

Into a 2 L flask equipped with a stirrer and a reflux condenser 1g (0.5 mol%) of tris(dibenzylydeneacetone)dipalladium(0) [Pd₂(dba)₃] is dissolved in 100 mL of tetrahydrofuran, then 2 g (1 mol%) of 2-dicyclohexylphosphino-2',6'-dimethoxybdipheyl (Sphos) added and the resulting solution stirred under a slight stream of argon for 30 min.

A solution of 3-(1-adamantyl)-4-methoxyphenylboronic acid, produced in the previous step and 61 g (0.23 M) of methyl 6-bromo-2-naphtenoate is added to the reaction mixture and stirred until dissolved. As the final component a solution of 64 g (0.6 M) of sodium carbonate in 400 mL of water, prepared in advance, is added at once. The process is carried on under vigorous stirring and reflux under a slight stream or argon for 4 h.

After the completion the reaction mixture is cooled to room temperature and filtered without separating the layers. The precipitate constitues the main portion of the product. The rest of the product is obtained by concentrating the organic layer to ¼, cooling and filtering off the precipitate.

The raw product is dissolved in 500 mL of dimethylacetamide, filtered hot, reduced to about 300 mL and left for 16 h at room temperature to crystallize. The crystals are filtered off and dried at 150-180 °C for 2 h. Methyl 6-[3-(1-adamantyl)-4-methoxyphenyl]-2-naphthenoate, 86 g, 65% based on the starting 2-(1-adamantyl)-4-bromoanisole. The purity of the product exceeds 98%.

Scaling the synthesis up 5 times under the optimal reaction conditions did not lower the yield of the end product.

### Step D: Basic hydrolysis of methyl 6-[3-(1-adamantyl)-4-methoxyphenyl]-2-naphtenoate.

### Example 18 (comparative example)

Into a 600 mL beaker 10 g of methyl-6-[3-(1-adamantyl)-4-methoxyphenyl]-2-naphthenoate is introduced, 300 mL of ethylene glycol added and heated almost to boiling. To the obtained clear solution 15 g of sodium hydroxide is added in 3 portions and vigorously stirred for 20 min. The hot reaction mixture is slowly added with vigorous stirring to cold 5% solution of aqueous hydrochloric acid. The suspension thus obtained is stirred for 30 min and filtered off. The precipitate is pressed on filter, washed with 3x500 mL of hot water. Dried at 100 °C for 16 h. 6-[3-(1-adamantyl)-4-methoxyphenyl]-2-naphtoic acid, 9.3 g (96%) with m.p. 320 °C and purity over 97% is obtained.
NMR (500 MHz, DMSO D₆
δ: 1.79 (s, 6H), 2.10 (s, 3H), 2.16 (s, 6H), 3.90 (s, 3H), 6.99 (d, 1H, J = 8Hz), 7.51 (s, 1H), 7.52 (d, 1H, J = 8 Hz), 7.76 (d, 1H, J = 9 Hz), 7.92 (d, 1H, J = 9 Hz), 7.98 - 8.02 (m, 3H), 8.55 (s, 1H), 12.7 (s, 1H).

Using 1,2-propanediol instead of ethylene glycol the concentration of the hydrolyzed methyl 6-[3-(1-adamantyl)-4-methoxyphenyl]-2-naphthenoate in solution can be increased by 50%.

### Example 19 (example according to the invention)

Into a 600 mL beaker 15 g of methyl-6-[3-(1-adamantyl)-4-methoxyphenyl]-2-naphthenoate is introduced, 300 mL of 1,2-propanediol added and heated almost to boiling. To the obtained clear solution 15 g of sodium hydroxide is added in 3 portions and vigorously stirred for 20 min. Further operations for treating the reaction mixture and separation of the end product are similar to those of Example 18. The yield of 6-[3-(1-adamantyl)-4-methoxyphenyl]-2-naphthoic acid is 96%.

Scaling up the synthesis 10 times under the optimal reaction conditions did not lower the yield and quality of the end product.

The disclosed process for manufacturing of adapalene is characterized by high yields of intermediates and end product, simple technology and possibility of scaling up.

## Claims

1. A process for manufacturing of 6-[3-(1-adamantyl)-4-methoxyphenyl]-2-naphthoic acid of formula (I) 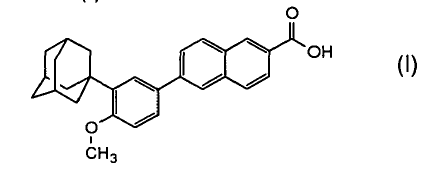 from a compond of formula (II) 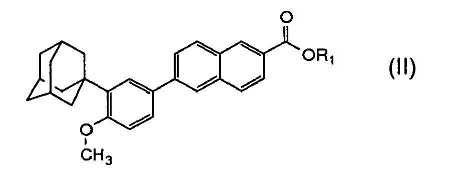 wherein R₁ is straight, branched or cyclic C₁-C₁₀alkyl, CH₂OR, wherein R is a C₁-C₅alkyl or -(CH₂)₀₋₁C₆H₅, **characterized in that** the process is performed by heating the starting compound with sodium or potassium hydroxide in 1,2-propylene glycol solution, with following acidification with inorganic acid and separation of the end product.

2. The process of claim 1, wherein the compound of formula (II) is prepared from compound of formula (III) 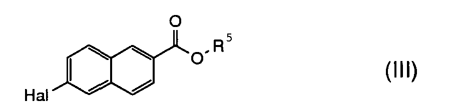 wherein R⁵ is K, Na, Li, H, straight C₁-C₁₀alkyl, branched C₃-C₁₀alkyl or cyclic C₃-C₁₀alkyl, CH₂OR, wherein R is a C₁-C₅alkyl or -(CH₂)₀₋₁C₆H₅, and Hal is Cl, Br or I, preferably Br,
by reacting with a compound of formula (IV) 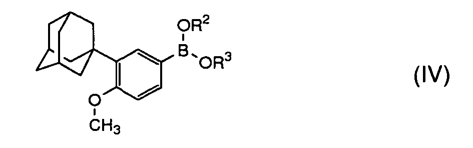 wherein R² and R³ independently of each other is K, Na, Li, H, straight C₁-C₁₀alkyl, branched C₃-C₁₀alkyl or cyclic C₃-C₁₀alkyl, CH₂OR, wherein R is a C₁-C₅alkyl or -(CH₂)₀₋₁C₆H₅, in presence of inorganic base and palladium catalyst or Pd[0] with or without a phosphine ligand.

3. The process of claim 2, wherein the source of Pd[0] is selected from the group, consisting of palladium acetate and tris(dibenzylydene-acetone)dipalladium(0).

4. The process of any claim 2 or 3, wherein the phosphine ligand is selected from a group consisting of 2-dicyclohexyphosphino-2',6'-dimethoxy-biphenyl, 2-dicyclohexylphosphino-2'-methylbiphenyl, 2-dicyclohexyl-phosphino-2',6'-dimethylbiphenyl, 2,2-dicyclohexylphosphinobiphenyl, 2-di-tert-butylphosphino-2'-methylbiphenyl, 2-di-*tert*-butylphosphino-2',6'-dimethylbiphenyl, 2-di-*tert-*butyl-phosphinobiphenyl, 2,4,6-tri-*iso*-propyl-2'-diphenylylphosphinobiphenyl, diphenylphosphinoferrocene, triphenyl-phosphine, tricyclohexylphosphine, tri-*tert*-butylphosphine, preferably 2-dicyclohexylphosphino-2',6'-dimethoxybiphenyl.

5. The process of any claim 2 or 3, wherein the palladium catalyst is tetrakis(triphenylphosphine)palladium(0).

6. The process of any claim from 2 to 5, wherein the inorganic base is selected from a group consisting of lithium, sodium, potassium and strontium carbonates, acetates and phosphates.

7. The process of any claim from 2 to 5, wherein the reaction is performed in the presence of organic solvents or mixture thereof or mixture of organic solvent with water.

8. The process of any claim of 2 to 7, wherein the palladium catalyst or Pd[0] and the compound of formula (IV) is used in a rate from 0.0005 to 0.1 mol of palladium catalyst to one mol of compound of formula (IV), preferably in a rate 0.005 mol to one mol.

9. The process of any claim of 2 to 8, wherein the reaction is performed at temperature from 20 °C to 100 °C.

10. The process of claim 2, wherein the compound of formula (IV) is prepared from a compound of formula (V) 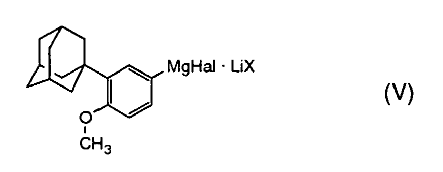 wherein Hal is Cl, Br or I, preferably Br,
X is Cl, Br, I, SO₄, ClO₄, BF₄, preferably Cl,
by reacting with a compound of formula B(OR⁴)₃, wherein R⁴ is a straight C₁-C₁₀alkyl, branched C₃-C₁₀alkyl or cyclic C₃-C₁₀alkyl, preferably CH₃.

11. The process of claim 10, wherein the reaction is performed at temperature from -70 °C to + 50 °C.

12. The process of claim 10 or 11, wherein the reaction is performed at temperature from 0 °C to + 5 °C.

13. The process of claim 10, wherein the compound of formula (V) is prepared from a compound of formula (VI) 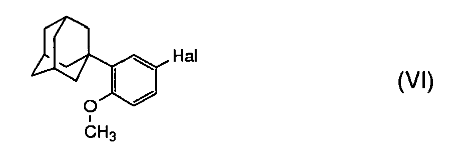 wherein Hal is Cl, Br or I, preferably Br,
by reacting with magnesium in aprotic inert solvent in the direct Grignard's reaction in the presence of an anhydrous inorganic lithium salt.

14. The process of claim 13, wherein the reaction is performed at a temperature from -70 °C to + 80 °C, preferably from 20 °C to 70 °C.

15. The process of claim 13 or 14, wherein the aprotic solvent in the reaction is tetrahydrofuran.

16. The process of any claim from 13 to 15, wherein the anhydrous inorganic lithium salt is selected from a group, consisting of anhydrous chloride, bromide, iodide, sulfate, perchlorate, tetrafluoroborate, preferably anhydrous lithium chloride.

17. The process of any claim from 13 to 16, wherein the anhydrous inorganic lithium salt is used in rate from 0.1 to 5.0 mol for one mol of compound (VI).

18. The process of any claim from 13 to 16, wherein the anhydrous inorganic lithium salt is used in the preferred rate from 1.2 to 1.5 mol for one mol of compound (VI).

19. The process of any claim from 2 to 9, wherein the compound of formula (IV) prepared by process of any claim from 10 to 18 is used without separation from the reaction mixture and purification.

## Patentansprüche

1. A Ein Verfahren für die Herstellung von 6-[3-(1-Adamantyl))-4-methoxyphenyl]-2-naphthoesäure der Formel (I) 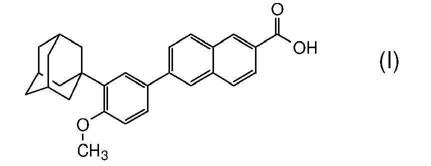 aus einer Verbindung der Formel (II) 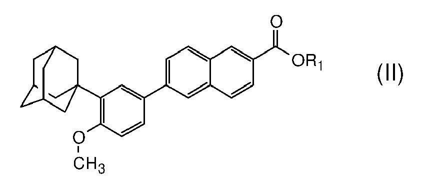 in der R₁ für geradkettig, verzweigt oder zyklisch C₁-C₁₀alkyl, CH₂OR steht, worin R für ein C₁-C₅alkyl oder -(CH₂)₀₋₁C₆H₅ steht, charakterisiert dadurch, dass das Verfahren durchgeführt wird, durch Erhitzen der anfänglichen Verbindung mit Natrium oder Kaliumhydroxid in einer Propylenglykol-Lösung mit der folgenden Versauerung durch anorganische Säure und Trennung des Endproduktes.

2. Das Vorgehen des Anspruches 1, in dem die Verbindung der Formel (II) vorbereitet wird aus der Verbindung der Formel (III) 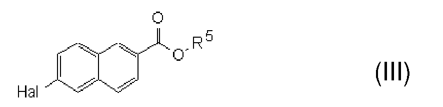 in der R⁵ K, Na, Li, H für geradkettig steht, C₁-C₁₀alkyl für verzweigt C₃-C₁₀alkyl oder zyklisch C₃-C₁₀alkyl, CH₂OR steht, wobei R steht für C₁-C₅akryl oder - (CH₂)₀₋₁C₆H_{5,} und Hal ist Cl, Br, oder I, vorzugsweise Br,
durch Reaktion mit einer Verbindung der Formel (IV) 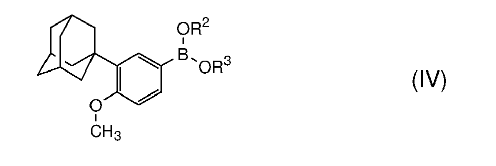 in der R² und R³ unabhängig voneinander stehen für K, Na, Li, H, geradkettig C₁-C₁₀alkyl, verzweigt C₃-C₁₀alkyl oder zyklisch C₃-C₁₀alkyl, CH₂OR, wobei R steht für C₁-C₅alkyl oder -(CH₂)₀₋₁C₆H₅, bei Vorhandensein einer anorganischen Basis und Palladium-Katalysatoren oder Pd[0] mit oder ohne einen Phosphin-Ligand.

3. Das Vorgehen für Anspruch 2, in dem die Source von Pd[0] aus der Gruppe gewählt wird, bestehend aus Palladiumazetat und Tris(dibenzylideneacetone)dipalladium (0) Produkt.

4. Das Vorgehen der Ansätze, entweder 2 oder 3, in denen das Phosphin-Ligand ausgewählt wird aus einer Gruppe, bestehend aus 2-dicyclohexyphosphino-2',6'-dimethoxy-biphenyl, 2-dicyclohexylphosphino-2'-methylbiphenyl, 2-dicyclohexyl-phosphino-2',6'-dimethylbiphenyl, 2,2-dicyclohexylphosphinobiphenyl, 2-di-*tert-*butylphosphino-2'-methylbiphenyl, 2-di-*tert*-butylphosphino-2',6'-dimethylbiphenyl, 2-di-*tert*-butyl-phosphinobiphenyl, 2,4,6-tri-*iso*-propyl-2'-diphenylylphosphinobiphenyl, diphenylphosphinoferrocene, triphenyl-phosphine, tricyclohexylphosphine, tri-*tert*-butylphosphine, vorzugsweise 2-dicyclohexylphosphino-2',6'-dimethoxybiphenyl.

5. Das Vorgehen der Ansätze 2 oder 3, in denen der Palladium Katalysator steht für Tetrakis(triphenylphosphine)palladium(0).

6. Das Vorgehen für alle Ansätze von 2 bis 5, in denen die organische Basis ausgewählt wurde aus einer Gruppe, bestehend aus Lithium, Natrium, Kalium und Strontium-Karbonaten, Azetaten und Phosphaten.

7. Das Vorgehen für alle Ansätze von 2 bis 5, in denen die Reaktion durchgeführt wird bei Vorhandensein von organischen Lösemitteln oder einer Mischung derselben oder einer Mischung von organischen Lösemitteln und Wasser.

8. Das Vorgehen von den Ansätzen 2 bis 7, in denen der Palladium Katalysator oder Pd[0] und die Verbindung der Formel (IV) verwendet wird in einem Verhältnis von 0.0005 bis 0.1 mol des Palladium-Katalysators zu der Verbindung der Formel (IV), bevorzugt ein einem Verhältnis von 0.005 mol zu einem mol.

9. Das Vorgehen für alle Ansätze von 2 bis 8, in denen die Reaktion stattfindet bei einer Temperatur von 20° C bis 100° C.

10. Das Vorgehen für den Anspruch 2, in dem die Verbindung der Formel (II) vorbereitet wird aus der Verbindung der Formel (V) 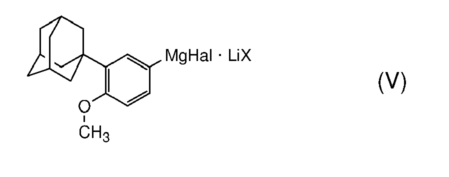 in dem Hal steht für Cl, Br oder I, vorzugsweise Br,
X steht für Cl, Br, I, SO₄, ClO₄, BF₄, vorzugsweise Cl,
mit Reaktionen mit einer Verbindung der Formel B(OR⁴)_{3,} in der R⁴ für ein gradkettiges steht, C₁-C₁₀alkyl verzweigt ist, C₃-C₁₀alkyl oder zyklisch C₃-C₁₀alkyl, vorzugsweise für CH₃ steht.

11. Das Vorgehen des Patentanspruchs 10, in dem die Reaktion herbeigeführt wird bei einer Temperatur von -70 ° C bis + 50 ° C.

12. Das Vorgehen des Patentanspruchs 10 oder 11, in dem die Reaktion herbeigeführt wird bei einer Temperatur von 0 ° C bis + 5 ° C.

13. Das Vorgehen des Patentanspruchs 10, in dem die Verbindung der Formel (V) vorbereitet wird aus der Verbindung der Formel (VI) 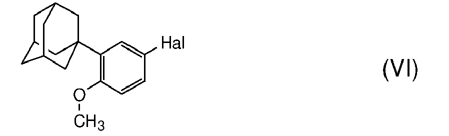 worin Hal steht für Cl, Br oder I, vorzugsweise Br,
durch Reaktion mit Magnesium in einem protonenfreien inerten Lösungsmittel in der direkten Grignard-Reaktion in Gegenwart von einem anhydrischen, anorganischen Lithiumsalz.

14. Das Vorgehen für Anspruch 13, in dem die Reaktion herbeigeführt wird bei einer Temperatur von -70° C bis + 80° C, vorzugsweise von 20° C bis 70° C.

15. Das Vorgehen für Anspruch 13 oder 14, worin das protonenfreie Lösemittel in der Reaktion Tetrahydrofuran (THF) ist.

16. Das Vorgehen für alle Ansprüche von 13 bis 15, bei denen das anhydrische, anorganische Lithiumsalz aus einer Gruppe gewählt wurde, bestehend aus anhydrischem Chlorid, Bromid, Jodid, Sulfat, Perchlorat, Tetrafluorborat , vorzugsweise einem anhydrischen Lithiumchlorid.

17. Das Vorgehen für alle Ansprüche von 13 bis 16, worin das anhydrische Lithiumsalz zur Verwendung kommt, in einem Verhältnis von 1,0 bis 5,0 Mol für eine Mol der Verbindung (VI)

18. Das Vorgehen für alle Ansprüche von 13 bis 16, worin das anhydrische Lithiumsalz zur Verwendung kommt, in einem Verhältnis von 1,2 bis 1,5 Mol für ein Mol der Verbindung (VI)

19. Das Vorgehen für alle Ansprüche von 2 bis 9, worin die Verbindung der Formel (IV), vorbereitet durch das Vorgehen für alle Ansprüche von 10 bis 18 wird verwendet ohne Trennung der Reaktionsmischung und der Reinigung.

## Revendications

1. Un procédé de fabrication d'acide 6-[3-(1-adamantyl)-4-méthoxyphényl]-2-naphthoïque de formule (I) 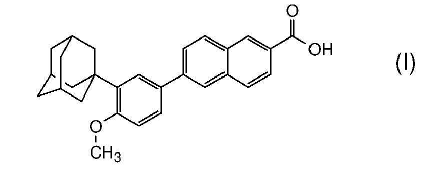 À partir d'un composé de formule (II) 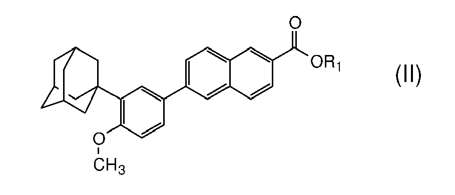 Dans la quelle R₁ un Alkyle C₁-C₁₀ linéaire, ramifié ou cyclique, CH₂OR, où R est un AlkyleC₁-C₅ ou -(CH₂)₀₋₁C₆H₅, **caractérisé en ce que** le processus est performé en chauffant le compose de départ avec du sodium ou de l'hydroxyde de potassium dans une solution de 1,2 de propylène de glycol, avec une acidification suivante d'acide inorganique et la séparation du produit final.

2. Le procédé de revendication 1, dans lequel le compose de formule (II) est préparé à partir du composé de formule (III) 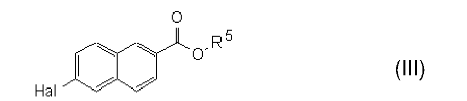 Dans lequel R⁵ est K, Na, Li, H, Alkyle C₁-C₁₀ linéaire, Alkyle C₃-C₁₀ ramifié ou Alkyle C₃-C₁₀ cyclique, CH₂OR, où R est un Alkyle C₁-C₅ ou -(CH₂)₀₋₁C₆H₅, et Hal est un Cl, Br ou I, de préférence Br,
En réaction à un compose de formule (IV) 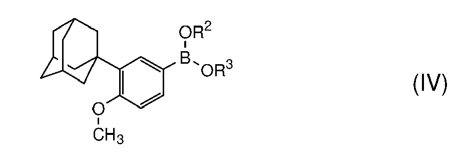 Dans lequel R² et R³, indépendamment des uns et autres, est un K, Na, Li, H, Alkyle C₁-C₁₀ linéaire, Alkyle C₃-C₁₀ ramifié ou Alkyle C₃-C₁₀ cyclique, CH₂OR, où R est un Alkyle C₁-C₅ ou -(CH₂)₀₋₁C₆H₅, en présence d'une base inorganique et d'un catalyseur de palladium ou Pd[0], avec ou sans de la phosphine de ligand.

3. Le procédé de revendication 2, dans lequel la source Pd[0] est sélectionnée à partir d'un groupement, constitué d'acétate de palladium et de tris(dibenzylydène-acétone)dipalladium(0).

4. Le procédé de l'une des revendications 2 ou 3, dans lequel la phosphine de ligand est sélectionné à partir d'un groupement constitué de 2-dicyclohexylphosphine-2',6'-diméthoxybiphényles, 2dicyclohexylphosphine-2'-méthyl-biphényle, 2-dicyclo-hexylphosphino-2',6'- diméthoxybiphényles, 2-2-dicyclohexylphosphine-biphényle, 2-di-tert-butylphosphino-2'- méthyl-biphényle, 2-di-tert-butyl-phosphino-2',6'- diméthylbiphényle, 2- di-tert-butylphosphino biphényle, 2,4,6-tri-iso-propyl-2'diphénylphosphino-phényle, diphénylphosphino ferrocène, triphénylphosphine, tricyclohexylphosphine, tri-tert-butylphosphine. Préférablement 2-dicyclohexylphosphine-2',6'-diméthoxybiphényles.

5. Le procédé de l'une des revendications 2 ou 3, dans lequel le catalyseur de palladium est le tétrakis(triphénylphosphine) de palladium(0).

6. Le procédé de l'une des revendications 2 ou 5, dans lequel la base inorganique est sélectionnée parmi un groupement constitué de lithium, de sodium, de carbonates de potassium et de strontium, d'acétates et de phosphates.

7. Le procédé de l'une des revendications 2 ou 5, dans lequel la réaction est effectuée en présence de solvants organique ou de leur mélange, ou d'un mélange de solvant organique avec de l'eau.

8. Le procédé de l'une des revendications 2 ou 7, dans lequel le catalyseur de palladium ou Pd[0] et le compose de formule (IV) est utilisé à un taux de 0,0005 à 0,1 mol du catalyseur de palladium pour une mol du composé de formule (IV), de préférence à un taux de 0.005 mol à une mol.

9. Le procédé de l'une des revendications 2 ou 8, dans lequel la réaction est effectuée à une température de 20°C à 100°C.

10. Le procédé de revendication 2, dans lequel le compose de formule (IV) est préparé à partir d'un composé de formule(V) 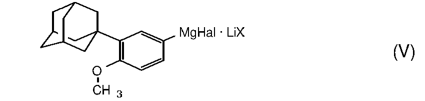 Dans lequel Hal est Cl, Br ou I, de préférence Br,
X est Cl, Br, I, SO₄, ClO₄, BF₄, de préférence Cl,
En réaction à un composé de formule B(OR⁴)_{3,} dans lequel R⁴ est un Alkyle C₁-C₁₀ linéaire, Alkyle C₃-C₁₀ ramifié ou Alkyle C₃-C₁₀ cyclique, de préférence CH₃.

11. Le procédé de revendication 10, dans lequel la réaction est effectué à une temperature de -70 °C à + 50 °C.

12. Le procédé de revendication 10 ou 11, dans lequel la réaction est effectué à une température de 0 °C à + 5 °C.

13. Le procédé de revendication 10, dans lequel le compose de formule (V) est préparé à partir d'un composé de formule (VI) 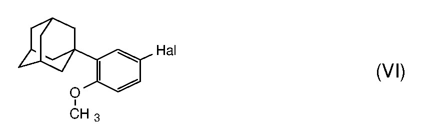 Dans lequel Hal est Cl, Br ou I, de préférence Br,
En réaction avec du magnésium avec du magnésium dans du solvant aprotique inerte, en relation directe aux organomagnésiens (réaction Grignard), en présence de sel de lithium anhydre inorganique.

14. Le procédé de revendication 13, dans lequel la réaction est effectué à une température de -70 °C à + 80 °C, de préférence de 20 °C à 70 °C.

15. Le procédé de revendication 13 ou 14, dans lequel le solvant aprotique de la réaction est tetrahydrofuran.

16. Le procédé de revendication de 13 à 15, dans lequel le sel de lithium anhydre inorganique est sélectionné à partir d'un groupement, constitué de chlorite anhydre, de bromure, d'iodure, de sulfate, de perchlorate, de tétafluoroborate, de chlorure de lithium de préférence anhydre.

17. Le procédé de revendication de 13 à 16, dans lequel le sel de lithium anhydre inorganique est utilisé à un taux de 0.1 à 5.0 mol pour un seul du composé (VI).

18. Le procédé de l'une des revendications 13 à 16, dans lequel le sel de lithium anhydre inorganique est utilisé à un taux 1.2 à 1.5 mol pour un seul du composé (VI).

19. Le procédé de l'une des revendications 2 à 9, dans lequel le composé de formule (IV) préparé par un procédé de l'une des revendications de 10 à 18 est utilisé sans séparation sans être séparé du mélange et de la purification de la réaction.
